# EUROPEAN PATENT APPLICATION

(11) **EP 0 681 791 A2**
(43) Date of publication of application: **15.11.1995**
(21) Application number: 95104512.9
(22) Date of filing: 27.03.1995
(51) Int. Cl.: A43B 7/02

(54) **Self-heating insole for the heating of feet**

(30) Priority: 31.03.1994 IT MI940242 U
(71) Applicant: Ronconi, Tania, I-24100 Bergamo (IT); Ronconi, Cinzia, I-24100 Bergamo (IT)
(72) Inventor: Ronconi, Tania, I-24100 Bergamo (IT); Ronconi, Cinzia, I-24100 Bergamo (IT)
(74) Representative: Di Iorio, Giuseppe, Dr.-Ing.

(57) **Abstract**

The shoe insole (1) is made-up of two sheets of PVC material welded with one another along its periphery (1A) and along some internal lines (2), preferably in longitudinal direction, with the aim of forming intercommunicating channels and chambers holding a chemical solution. One of these chambers holds a metallic plate (4), that, after a manual handling, such as friction, made by the user, primes a chemical reaction in the solution that occurs with heat development.

The insole is suitable for any type of shoes, particularly for sport shoes (ski-boots and après-ski outfits, fishing boots, hunting boots and riding boots) and, after the priming of the chemical reaction in the solution, allows to heat the foot of the user.

## Description

This invention refers to the field of heating devices and particularly to the ancillary equipments foreseen for the shoes suitable for heating the people feet.

The invention is advantageously used to make insoles for shoes of general use and particularly for sport shoes.

According to the invention, the insole is made-up of two sheets of PVC material suitably welded with one another to make intercommunicating channels and chambers in its internal volume.

The channels and chambers hold a non-toxic nor noxious chemical solution, made-up of a water solution of alkaline salts of carboxylic acids.

Further a chamber holds in its internal volume a metallic element, such as a steel plate according to the standard UNI 6900/71, the used steel being corresponding to the denomination X12CrNi17.07 (AIBI 301).

By slightly flexing or rubbing the metallic element it is caused the priming of a chain reaction in the molecules of the chemical solution, and this reaction, starting from the chamber holding the metallic element, propagates in all channels and chambers of the insole, causing the solution to develop heat, and so the solution can reach a temperature of ab. 50 centigrade degrees. Once the chemical reaction is primed, the insole is ready to be inserted into the shoe, where, being in contact with user's foot, shall keep the constant temperature for ab. 2-3 hours.

It is preferably made an insole that shows some welding lines that form longitudinal channels and chambers communicating with one another and holding the above mentioned chemical solution.

Further characteristics and advantages of the invention shall be evident from the following description referred to a typical embodiment thereof, shown in the annexed drawings, in which:
Figure 1 shows the insole or envelope with weldings and longitudinal, rectilinear and parallel connecting channels;
Figure 2 shows a second embodiment of the insole with connection channels in the form of fish-bone, and
Figure 3 shows, as unlimiting example, the position in which the heating insole is inserted in the internal volume of a shoe (ski-boot).

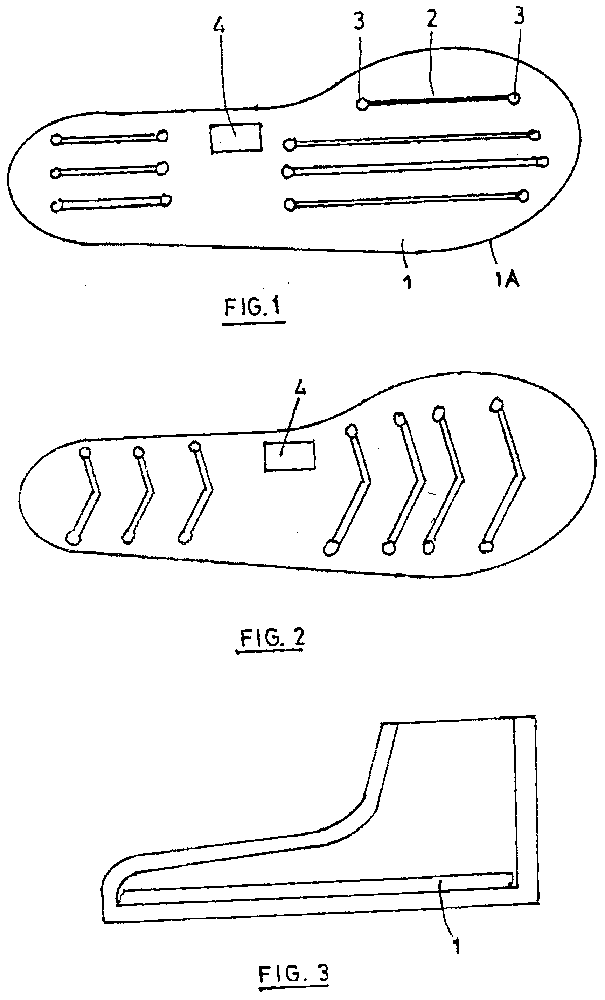

The Figure 1 shows a first embodiment of an insole 1 made-up of two sheets of PVC material or other suitable material of inflatable type, joined by high-frequency thermal welding or by ultrasound welding.

The welding is made both along the periphery 1A of the insole and along the internal lines 2 or in circular areas 3 or with other shapes with the aim of establishing, within said insole, some channels and chambers that are filled with a water solution of alkaline salts of carboxylic acids.

This solution is of the type suitable to cause a chemical reaction with heat development. Further one of the chambers holds a metallic element for priming the above mentioned chemical reaction.

The internal welding lines are preferably made in longitudinal direction, in rectilinear form and parallel with one another to form similar channels therebetween. They are made both on the front part and on the rear part of the insole in such a manner to cause the heat generated by the reaction to involve the whole insole and then it heats the whole foot surface.

The Figure 2 shows the welding lines that are made under the form of fish-bone, but they can have also other forms according to the type of shoe use. For example, they can be made in oblique form, in circumference arc or coil forms and this is made in relation to the type of activity the user must make, for example of sport type (mountain climbing boots, ski-boots or long distance ski-race boots) or in normal use (walk shoe).

The Figures 1 and 2 show a metallic element 4 made under the form of a plate, having an incision or not having an incision, but that can also have the form of a little coil, preferably enclosed in a tubular sheath for protecting the external envelope. The plate or metallic coil is inserted in a chamber made in the central part of the insole in conjuction with the central zone of the user's foot.

The metallic element is submitted to handling by the user for causing the priming of chemical reaction in the solution, in which it is embodied, and then the heating of the insole.

The Figure 3 shows an illustrative example of the use of the insole in a mountain boot, both of ski type and climbing type, in which the insole is put on the internal bottom of the boot just after the priming of chemical reaction.

The device use is very simple. When the user wishes to heat the foot, he seizes the insole and by a rubbing, plying, flexing or friction of the plate or coil causes the priming of the chemical reaction that occurs with heat development causing in this manner the insole heating. Usually the chemical solution is made-up of sodium acetate and distilled water, preferably in the percentages of 46% by weight of water and 54% by weight of sodium acetate.

The heated insole, after its insertion in the boot, supplies a comfort sensation (hot feeling) to the foot and re-establishes the circulation of the blood. The device can be also used in other fields, for example in the form of a jacket to be worn under the mountain wind-cheater, or it can be used near body parts sensible to cold, such as back bottom (glutaei) or legs or calves.

It can be also used as heating cushion or inserted in the slippers or in rest shoes or in ski gloves. It is clear that according to the use it shall assume the more suitable anatomic form, but it shall have, in any case, some welding lines that form internal inflatable spaces, holding the water solution and the metallic element for activating the reaction. This element can be made, more than of stainless steel, also of copper or aluminium alloy or other suitable material on condition that it is suitable to cause, by its handling, the above mentioned chemical reaction.

The reaction can be also quickened putting at first the insole near a heat source, for example a stove in a mountain refuge or immersing it in hot water. In any case, after having started the reaction, the insole reaches immediately a temperature near to 50 centigrade degrees and its cooling occurs within a reasonably long time of 2-3 hours.

During the cooling the water solution hardens, but the insole can be further used as heating means after having introduced it, over some minutes, in a container holding boiling water. After this treatment, really, the insole gets again the start conditions, that is, it is ready to be used another time and so on.

A further advantage of the insole according to the invention is provided as it can be used as a damper of vibration caused during the user's deambulation or sport activity.

Finally the insole of PVC sheets can have external knurled surfaces with anti-slipping properties.

## Claims

**1.** An insole to be inserted in a shoe, particularly a shoe for sport use, characterized in that it is made-up of a plate holder or envelope (1) made-up of inflatable material, whose internal volume holds both a chemical solution suitable for causing a reaction with heat development when it is activated, and a metallic element (4) for activating this reaction when said element is submitted to user's handling, such as pressure, flexing, bending, rubbing or friction.

**2)** An insole according to claim 1, characterized in that the envelope (1) is made-up of two sheets coupled with one another for establishing in its internal volume some intercommunicating channels and chambers and holding the chemical solution, one of said chambers holding the metallic element (4) used to activate the chemical reaction.

**3)** An insole according to claims 1 and 2, characterized in that the chemical solution is made-up of a water solution of alkaline salts of carboxylic acids.

**4)** An insole according to claims 1 and 2, characterized in that the chemical solution is made-up of sodium acetate and distilled water, preferably in the percentages of 46% by weight of water and 54% by weight of sodium acetate.

**5)** An insole according to claims 1 and 2, characterized in that the metallic element is made-up of a plate (4) of steel according to standard UNI 6900/71 with steel corresponding to the denomination X 12 Cr Ni 17.07 (AISI 301) or by a metallic coil preferably closed in a tubular sheath for protecting the external envelope.

**6)** An insole according to claims 1 and 2, characterized in that the envelope is made-up of two sheets of PVC material calendered to be hand-soft, thermally welded by high frequency or by ultrasounds both on the periphery (1A) and along internal welding lines (2) that form the above mentioned channels and chambers.

**7)** An insole according to claim 6, characterized in that the welding lines have a longitudinal behaviour, are rectilinear and parallel (2) with one another for establishing corresponding channels among one another.

**8)** An insole according to claim 6, characterized in that the welding lines have an oblique form or a fish-bone form, or circumference arc or coil forms.

**9)** An insole according to claims from 6 to 8, characterized in that the welding lines are made both in conjuction with the front part and the rear part of the insole and the internal chamber holding the metallic element is made in the central part of the insole.

**10)** An insole according to preceding claims, made-up of PVC sheets having external knurled surfaces with anti-slipping properties.
